# EUROPEAN PATENT SPECIFICATION

(11) **EP 2 948 187 B1**
(45) Date of publication and mention of the grant of the patent: **16.10.2019**
(21) Application number: 14708640.9
(22) Date of filing: 23.01.2014
(51) Int. Cl.: A61K 51/04, A61K 31/4155, A61K 31/42, C07D 261/08, C07D 261/14, C07D 405/04, A61P 35/00, A61P 29/00

(54) **HETEROCYCLES AND THEIR RADIOLABELED ANALOGS USEFUL AS COX-1 SELECTIVE INHIBITORS**
HETEROCYCLEN UND DEREN RADIOMARKIERTEN ANALOGA ALS SELEKTIVE COX-1-INHIBITOREN
HÉTÉROCYCLES ET LEURS ANALOGUES RADIOMARQUÉS UTILES EN TANT QU'INHIBITEURS SÉLECTIFS DE COX-1

(30) Priority: 24.01.2013 US 201361756147 P
(43) Date of publication of application: 02.12.2015
(73) Proprietor: Universita' Degli Studi di Bari, 70121 Bari (IT)
(72) Inventor: SCILIMATI, Antonio, 70125 Bari (IT); PERRONE, Maria, Grazia, 70125 Bari (IT); VITALE, Paola, 70125 Bari (IT)
(74) Representative: Trupiano, Federica
(86) International application number: PCT/IB2014/000064
(87) International publication number: WO 2014/115020

(56) References cited:
- WO-A1-03/026652
- US-A- 5 478 856
- US-A- 5 478 856
- GOTO K ET AL: "Analgesic effect of mofezolac, a non-steroidal anti-inflammatory drug, against phenylquinone-induced acute pain in mice - A new antiinflammatory agent, selectively inhibits prostaglandin G/H synthase/cyclooxygenase (COX-2) activity in vitro", PROSTAGLANDINS, BUTTERWORTH, STONEHAM, MA, US, vol. 56, no. 4, 1 July 1998 (1998-07-01), pages 245-254, XP004146553, ISSN: 0090-6980, DOI: 10.1016/S0090-6980(98)00054-9
- MCCARTHY T J ET AL: "Radiosynthesis, in vitro validation, and in vivo evaluation of <18>F-labeled COX-1 and COX-2 inhibitors", THE JOURNAL OF NUCLEAR MEDICINE, SOCIETY OF NUCLEAR MEDICINE, US, vol. 43, no. 1, 1 January 2002 (2002-01-01), pages 117-124, XP002596321, ISSN: 0161-5505
- MARIA GRAZIA PERRONE ET AL: "Diarylheterocycle Core Ring Features Effect in Selective COX-1 Inhibition", CHEMMEDCHEM, vol. 7, no. 4, 25 January 2012 (2012-01-25), pages 629-641, XP055110378, ISSN: 1860-7179, DOI: 10.1002/cmdc.201100530 cited in the application
- MCCARTHY T J ET AL: "Radiosynthesis, in vitro validation, and in vivo evaluation of <18>F-labeled COX-1 and COX-2 inhibitors", JOURNAL OF NUCLEAR MEDICINE, SOCIETY OF NUCLEAR MEDICINE, RESTON, VA, US, vol. 43, no. 1, 1 January 2002 (2002-01-01), pages 117-124, XP002596321, ISSN: 0161-5505
- DI NUNNO ET AL: "Novel Synthesis of 3,4-Diarylisoxazole Analogues of Valdecoxib: Reversal Cyclooxygenase-2 Selectivity by Sulfonamide Group Removal", JOURNAL OF MEDICINAL CHEMISTRY, AMERICAN CHEMICAL SOCIETY, US, vol. 47, no. 20, 1 January 2004 (2004-01-01), pages 4881-4890, XP002335576, ISSN: 0022-2623, DOI: 10.1021/JM040782X
- GOTO K ET AL: "Analgesic effect of mofezolac, a non-steroidal anti-inflammatory drug, against phenylquinone-induced acute pain in mice - A new antiinflammatory agent, selectively inhibits prostaglandin G/H synthase/cyclooxygenase (COX-2) activity in vitro", PROSTAGLANDINS, BUTTERWORTH, STONEHAM, MA, US, vol. 56, no. 4, 1 July 1998 (1998-07-01), pages 245-254, XP004146553, ISSN: 0090-6980, DOI: 10.1016/S0090-6980(98)00054-9

## Description

### Technical background

COX-1 isoenzyme plays a significant role in a variety of diseases, as it catalyzes the bioprocesses behind many health problems. The belief that cyclooxygenase-1 (COX-1) inhibition is the root of side effects associated with traditional nonsteroidal antiinflammatory drugs (tNSAIDs) has been abandoned over the last decade, and indeed, the COX-1 isoenzyme has been receiving increased attention as a pharmacotherapeutic target because of its involvement in many health problems. With better knowledge about the extent of COX-1 expression in many cells and tissues, it has been possible to gain significant insight into its role in atherosclerosis, endothelial dysfunction, neuro-inflammation, inflammatory syndrome, pain and pain processing, pre-term labor, a number of types of cancer, and gastrointestinal toxicity. Prostaglandins (PGs), as main products of the arachidonic acid bio-conversion catalyzed by COXs (COX-1 and COX-2), are thought to be determinants in the protection of gastric mucosa, and COX-1 expression levels in the GI tract are much higher than those of the other cyclooxygenase isoform, COX-2. Stomach irritation is one of the major side effects of NSAIDs, and it is thought to be caused by the inhibition of COX-1. However, various experiments have suggested that COX-1 deficiency or inhibition is compatible with normal small intestine integrity, without evidence of COX-1-selective inhibition being the cause of gastric damage. Therefore, highly selective COX-1 inhibitors could be useful pharmacological tools for shedding light on the role of COX-1 in the damage of gastric tissues. The development of selective COX-1 inhibitors could be quite relevant for the treatment of diseases in which the COX-1 isoform is negatively overexpressed. Very few selective COX-1 inhibitors are currently known.

The inventors have been publishing the results of an investigation aimed to identify the molecular determinants that switch the inhibitory activity toward the COX-1 isoenzyme and away from COX-2. These data were partly reported in Chem. Med. Chem. 2012, 7, 629 - 641, firstly published on the web on January 25, 2012.

Goto K et al. Prostaglandins, Butterworth, Stoneham, MA, US, vol.56, no. 4, 1 July 1998, pages 245-254, discloses mofezolac ([3,4-di(4-methoxyphenyl)-5-isoxazolyl] acetic acid) and its use in the treatment of inflammation and pain.

It was surprisingly found out that among a number of compounds which were synthesized and tested, only compound 5-(furan-2-yl)-1-(4-methoxyphenyl)-3-(trifluoromethyl)-1H-pyrazole **(I)** was found to be particularly active in the selective inhibition of COX-1 [COX-1 IC₅₀ = 3.4 mM; COX-2 IC₅₀ > 100 mM]. After said publication, two further compounds were prepared, i.e. the hydroxyl derivative of compound (**I**), herein after referred to as compound (**II**). The invention is defined by the claims.

Disclosed herein but not forming part of the present invention are compounds ¹²C-(**I**), (**II**), ¹¹C-(**I**) and their salts.

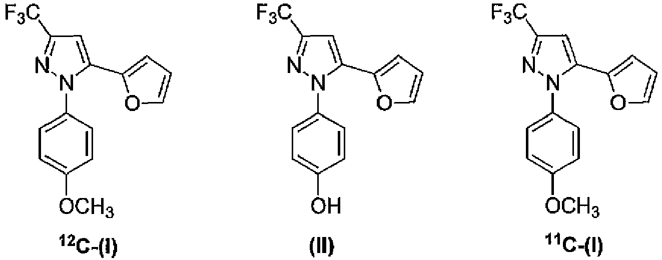

The salts of the compounds of formula ¹²C-(**I**) and ¹¹C-(**I**) may be pharmaceutically acceptable salts, in case they are used in pharmaceutically acceptable compositions. However, the salts of compounds of formula ¹²C-(**I**) and ¹¹C-(**I**) may also be non-pharmaceutically acceptable, in case they should be used for chemical purposes, or as a tool for "in vivo" tests to study the physiopathological role of COXs. Compounds of formula ¹²C-(**I**) and ¹¹C-(**I**) may be prepared according to the reaction details given in the experimental section of this application.

The biological assays were carried out as disclosed in Chem. Med. Chem. 2012, 7, 629 - 641.

Also disclosed herein but not forming part of the present invention are radiolabeled compounds of formula (**I**) or one of its salts wherein one or more of the atoms is a ¹¹C carbon atom and/or a ¹⁸F atom as a radionuclide.

Preferably, the ¹¹C carbon atom in the compound of formula ¹¹C-(**I**) is the carbon atom of the methoxy group.

The ¹¹C and/or ¹⁸F radiolabelled isotopic derivatives are especially useful in diagnostics, namely in Positron Emission Tomography (PET) diagnostic method. Recent reports have shown that the expression of COX-1 is an early event of several human pathologies including neuro-inflammation, inflammatory syndrome, neurodegenerative diseases, atherosclerosis, endothelial dysfunction preterm labor, pain, and carcinogenesis. In particular, COX-1 is overexpressed in various stages and progression of human epithelial ovarian cancers, where it controls the production of prostaglandins and promotes angiogenic growth factor production. A significantly reduction of tumor growth was found in an *in vivo* model with ovarian surface epithelial cells allografted in female nude mice, after treatment with SC-560, a highly selective COX-1 inhibitor. COX-1 is an ideal target, as a novel biomarker, for the diagnosis by imaging and the treatment of epithelial ovarian cancer in humans. Unfortunately, treatment of this neoplastic diseases is still limited because of the lack of a non-invasive and effective imaging agent for early diagnosis. So, development of a COX-1 -targeted Positron Emission Tomography (PET) radiotracer is a matter of the extreme importance.

Also disclosed herein but not forming part of the present invention is a diagnostic method for the detection and the examination of neuro-inflammation and cancer, especially ovarian cancer, which comprises the administration of a ¹¹C and/or ¹⁸F radiolabeled compound of formula (I) or of one of their salts to the mammal to be examined.

Preferably, the ¹¹C-radiolabeled compound is that of formula ¹¹C-(**I**) bearing the ¹¹C carbon atom in the methoxy group.

Said compound may be prepared according to any conventional method, for instance by replacing the group containing the ¹²C carbon atom of the compound of formula ¹²C-(**I**) with a ¹¹C carbon atom as a radionuclide. An illustrative, detailed reaction is reported in the experimental section of this application.

According to the present invention, the mammal is preferably a human being. The present disclosure further relates to a pharmaceutical or a diagnostic composition comprising the compound of formula ¹¹C-(**I**), a radiolabeled compounds thereof or a pharmaceutically acceptable salts thereof.

Also disclosed herein but not forming part of the present invention is a process for the preparation of compound of formula ¹²C-(**I**) and the radiolabeled compound ¹¹C-(**I**), thereof or a pharmaceutically acceptable salt thereof, as disclosed in the experimental section of the application.

Also disclosed is the process of demethylation and re-methylation of a mofezolac precursor (**III**), as well as its conversion into compounds (**IV**), ¹¹C-(**III**), (**V**) and (**VI**) (Figure 2, Scheme 1). Both the mofezolac (**VII**) (Scheme 2) and its precursor (**III**) are COX-1 selective inhibitors (COX-1 IC₅₀ values are 29 and 76 nM, respectively).

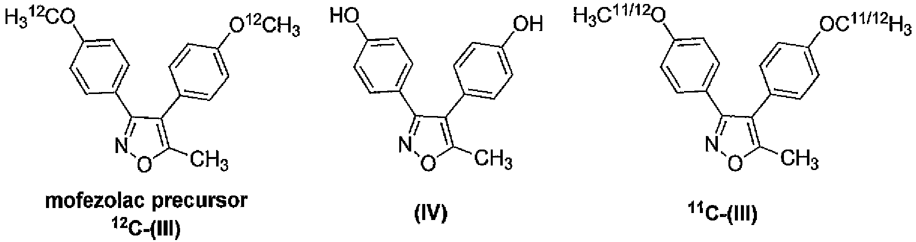

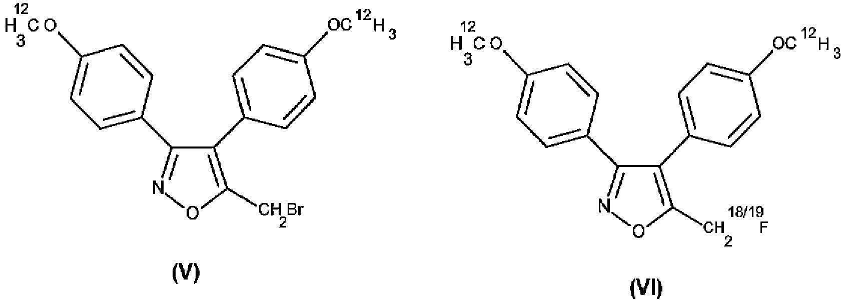

The re-methylation reaction is accomplished to mimic the incorporation into the mofezolac precursor ¹²C-(**III**), of the radio-isotope ¹¹C to obtain ¹¹C-(**III**), able to emit positrons. The methyl bonded to the isoxazole C₅ of mofezolac precursor (**III**) was brominated to compound **(V),** and the bromide replaced with ¹⁹F [compound **(VI),** step d, Scheme 1] to mimic the incorporation of the radio-isotope ¹⁸F, a positron emitter.

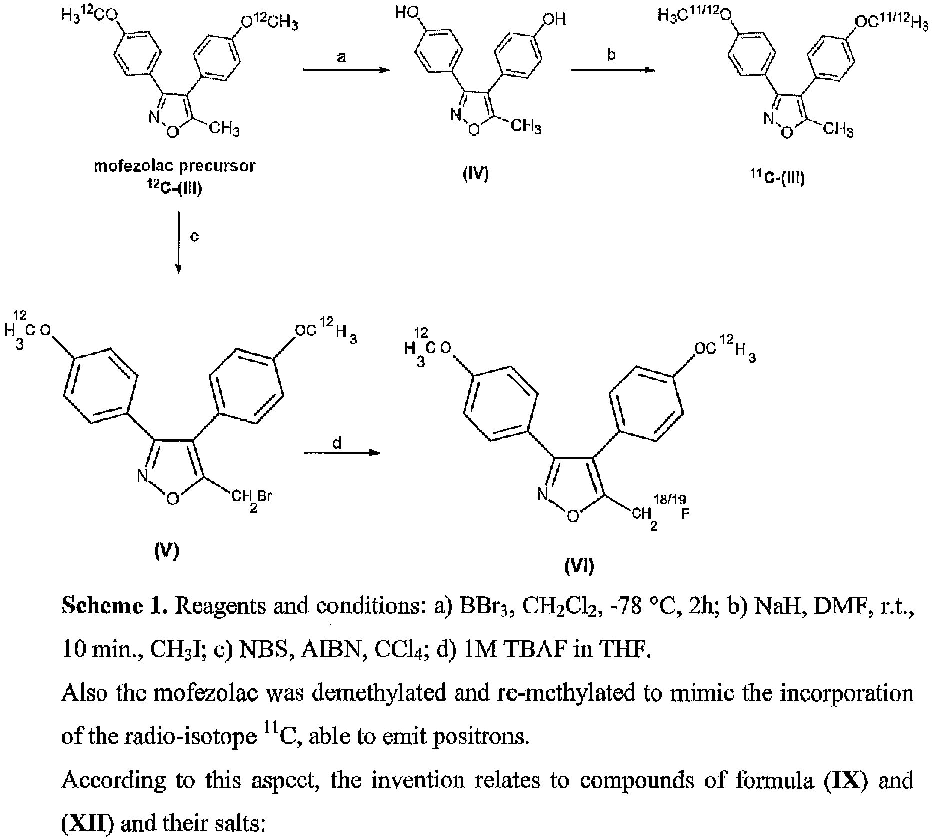

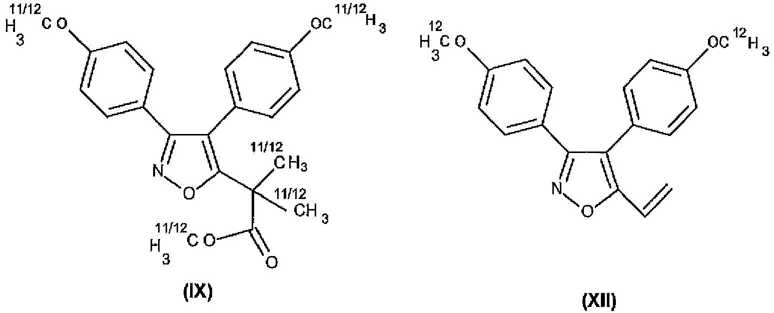

The invention discloses a process for the preparation of compound of formula ^{11/12}C-(**IX**) and compound (**XII**), thereof or a pharmaceutically acceptable salt thereof, as disclosed in the experimental section of the application.

According to another of its aspects, the invention relates to a pharmaceutical or a diagnostic composition comprising the compound of formula ¹¹C-(**IX**), a radiolabeled compounds thereof or a pharmaceutically acceptable salts thereof.

Accordingly, the present invention also relates to a ¹¹C radiolabeled compound of formula (IX) or one of its salts for use in a diagnostic method for the detection and the examination of neuro-inflammation and cancer, especially ovarian cancer, which comprises the administration of said ¹¹C radiolabeled compound of formula (**IX**) or of one of its salts to the mammal to be examined.

As another preferred embodiment, the invention relates to compounds of formula ^{11/12}C-(IX) and (XII) or pharmaceutically acceptable salts thereof for use in a method for preventing and/or treating a pathology selected from cancer, neuro-inflammation, inflammatory syndrome, cardioprotection, fever and pain which comprises administering to a mammal in need thereof an effective amount of the compounds of formula ^{11/12}C-(**IX**) and (**XII**) or pharmaceutically acceptable salts thereof.

Besides, the mofezolac carboxylic acid moiety was reduced to alcohol, that in turn was activate as tosylate(Ts)-compound (**XI**) and by treatment with Bu₄NF afforded the vinylisoxazole (**XII**).

In particular compounds were prepared as depicted in the following Scheme 2:

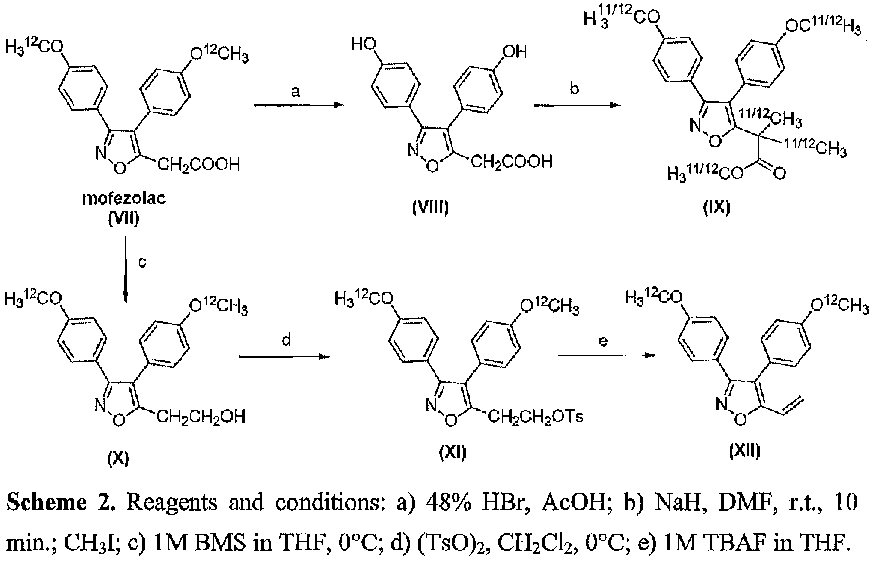

Also disclosed herein but not forming part of the present invention are compounds of formula (**XIII**), (**XIV**), and their salts

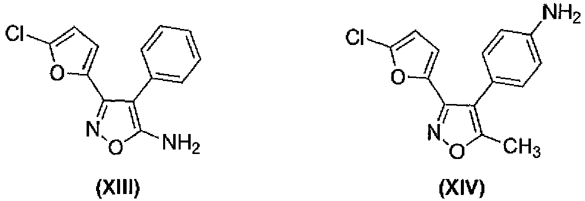

In particular, the 3-(5-chlorofuran-2-yl)-4-phenylisoxazol-5-amine (**XIII**) was found to be highly potent and selective COX-1 inhibitor (IC₅₀ =1.1 µM). It was prepared by 1,3-dipolar cycloaddition with 40% yield by a one pot procedure, from 5-chlorofuran-2-carbonitrile oxide and lithiated phenylacetonitrile as dipolarophile (Scheme 3).

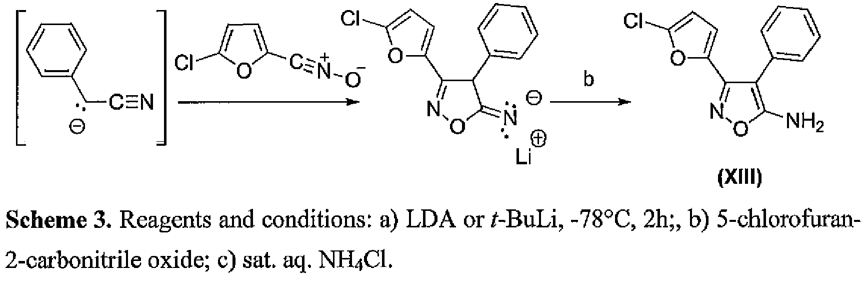

The 3-(5-chlorofuran-2-yl)-4-(4-aminophenyl)-5-methylisoxazole (**XIV**) (COX-1 IC₅₀=4.3 µM) was prepared, to verify the influence on COX-1 activity of the amine group on phenyl at isoxazole-C4 (XIII), starting from the inactive 3-(5-chlorofuran-2-yl)-4-(4-nitrophenyl)-5-methylisoxazole (XV):

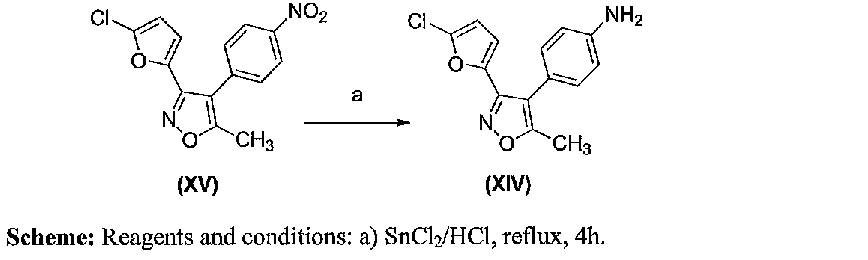

Compounds selected from compounds of formula (V), (VI), (IX) and (XII) and their salts, especially their pharmaceutically acceptable salts, are novel compounds and represent a subject-matter of the invention, as well their use for preventing and/or treating a COX-1-related patholgy and for preventing and/or treating a pathology selected from cancer, neuro-inflammation, inflammatory syndrome, cardioprotection, fever and pain.

According to another of its aspects, the invention relates to a pharmaceutical or a diagnostic composition comprising the compounds of formula (V), (VI), (IX) and (XII) or their pharmaceutically acceptable salts or radiolabeled compounds thereof or pharmaceutically acceptable salts thereof.

Radiolabeled derivatives of the compounds of formula (V), (VI), (IX), and (XII) and their salts, especially their pharmaceutically acceptable salts are novel compounds and represent a subject-matter of the invention, as well their use in radiomethabolic therapy and cancer, especially ovarian cancer and neuroinflammation diagnosis. Particularly preferred compounds are selected from those of formula ¹⁸F-(VI) and ¹¹C-(IX) and their salts.

The processes disclosed in this application for preparing the above compounds are also a subject-matter of the invention.

The experimental section of the application illustrates the invention.

### Experimental Section

### Reference Example 1

### 5-(Furan-2-yl)-1-(4-methoxyphenyl)-3-(trifluoromethyl)-1H-pyrazole (I):

4-Methoxyphenylhydrazine (0.669 gr, 4.85 mmol) was added dropwise to 4,4,4-trifluoro-1-(furan-2-yl)butane-1,3-dione (1 g, 4.85 mmol) and Al₂O₃ (1.484 g, 14.55 mmol). The heterogeneous reaction mixture was stirred at room temperature for 28 h. CH₂Cl₂ was then added, the suspension was filtered over silica gel, and the solvent was removed under reduced pressure. Column chromatography (silica gel, PE/EtOAc 7:3) of the reaction crude afforded 5-(furan-2-yl)-1-(4-methoxyphenyl)-3-(trifluommethyl)-*1H*-pyrazole (**I**) (530 mg, 36% yield) as an orange solid; mp: 71.0-73.0 °C); ¹H NMR (400 MHz, CDCl₃): δ=7.43-7.42 (m, 1H, furyl proton), 7.37-7.34 (m, 2H, aromatic protons), 7.00-6.98 (m, 2H, aromatic protons), 6.89 (s, 1H, pyrazole proton), 6.34-6.32 (m, 1H, furyl proton), 5.91-5.89 (m, 1H, furyl proton), 3.88 ppm (s, 3H, OMe); ¹⁹F NMR (376 MHz, CDCl₃): δ=-66.5 ppm; ¹³C NMR (100 MHz, CDCl₃, δ): 175.0, 160.5, 143.6 (m), 143.3, 136.6, 132.6, 127.7, 127.3, 121.3 (q, ¹J_{C-F}=268 Hz), 110.5, 109.8, 103.5, 55.8 ppm; FTIR (KBr): v=2963, 2920, 2849, 1609, 1505, 1461, 1431, 1386, 1301, 1245, 1214, 1178, 1155, 1125, 1022, 973, 832, 801, 744 cm⁻¹; GC-MS (70 eV) *m*/*z* (rel. int): 308 (100), 293 (10), 265 1, 254 (5), 237 (4), 217 (4), 196 (3), 185 (4), 168 (3), 115 (2), 103 (2), 90 (3), 77 (7), 64 (7), 51 (3); Anal. calcd for C₁₅H,₁₁F₃N₂O₂: C 58.45, H 3.60, N 9.09, found: C 58.43, H 3.65, N 9.10.

### Example 2

The compounds below which are not covered by the claims are for reference only. The compounds may be prepared according to the following scheme:

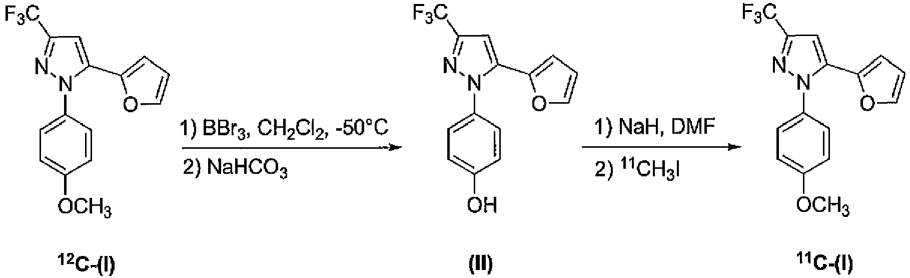

**4-[3-(Trifluoromethyl)-5-(furan-2-yl)-*1H*-pyrazol-1-yl]phenol** (**II**). 1 mL of Boron tribromide solution 1.0 M in methylene chloride was added to a solution of 5-(Furan-2-yl)-1-(4-methoxyphenyl)-3-(trifluoromethyl)-*1H*-pyrazole (**I**) (100mg, 0.32mmol) in CH₂Cl₂ dry (5 mL) at -50°C. The reaction mixture was stirred at room temperature for 18h; NaHCO₃ was then added, and after separation of the two phases, the organic layer was dried over Na₂SO₄ and the solvent was removed under reduced pressure. Column chromatography (silica gel, PE/EtOAc 8:2) of the reaction crude afforded **4-[3-(trifluoromethyl)-5-(furan-2-yl)-*1H*-pyrazol-1-yl]phenol (II)** (90 mg, 96% yield). ¹H NMR (300 MHz, CDCl₃): δ=7.53-7.52 (m, 1H, furyl proton), 7.24-7.20 (m, 2H, aromatic protons), 6.95-6.90 (m, 3H, 2H aromatic protons and 1H pyrazole proton), 6.38-6.37 (m, 1H, furyl proton), 5.94-5.92 (m, 1H, furyl proton); ¹³C NMR (100 MHz, CDCl₃): δ=156.1, 140.6, 140.3, 139.6 (q, ²J_{C-F}= 38 Hz), 134.2, 128.1, 124.7, 118.5 (q, ¹J_{C-F}=268 Hz), 112.8 (m), 108.3, 106.8 (m), 99.5 (m) ppm; ¹⁹F NMR (376 MHz, CDCl₃): δ= -62.8 ppm; FT IR (KBr): v=3169, 2925, 2853, 1612, 1594, 1524, 1507, 1446, 1434, 1385, 1290, 1247, 1221, 1177, 1157, 1142, 1104, 1030, 997, 976, 839, 805, 748 cm-1; GC-MS (70 eV) *m*/*z* (rel. int): 294 (100), 275 (8), 265 (14), 248 (6), 240 (8), 217 (2), 197 (6), 171 (6), 115 (3), 90 (1), 77 (2), 65 (10), 52 (3).

**5-(Furan-2-yl)-1-[4-(¹¹C)methoxyphenyl]-3-(trifluoromethyl)-*1H*-pyrazole [¹¹C-(I)]**. NaH (180 mg; 7.5 mmol) was added to a solution of 4-[3-(trifluoromethyl)-5-(furan-2-yl)-*1H*-pyrazol-1-yl]phenol (**II**) (30 mg; 0.10 mmol) in 5 mL of DMF dry. The mixture was stirred for 10 minutes and then ¹¹CH₃I (0.1 mL, 1.6 mmol) was added. The reaction mixture was then kept under reflux for 1 h. After cooling, EtOAc was added, and the mixture was washed for three times with brine, and after separation of the two phases, the organic layer was dried over Na₂SO₄, and the solvent was removed under reduced pressure. The reaction afforded an orange solid (32 mg, 98% yield).

### Preparation of 3,4-bis(4-hydroxyphenyl)-5-methylisoxazole (IV)

To a solution of **(III),** [3,4-bis(4-methoxyphenyl)-5-methylisoxazole] (100 mg, 0.34 mmol) in CH₂Cl₂ (5 mL) kept at -78 °C by a bath of dry ice/acetone, 1M BBr₃ in CH₂Cl₂ was added dropwise (1 mL, 1 mmol). The reaction mixture was stirred for 2 h, allowing the temperature to reach r.t., Then, NaHCO₃ sat. aq. solution was added to the reaction mixture. The aqueous solution was extracted with CH₂Cl₂, the organic layer was dried over anhydrous Na₂SO₄ and the solvent removed under reduced pressure. The product **2** was isolated as a white solid (45% yield) by chromatography on silica gel (hexane/ethyl acetate = 6:4) of the reaction crude. Its structure was confirmed by ¹H NMR (DMSO-*d₆*) δ: 2.40 (3H, s), 6.75 (2H, d, *J=* 8.8 Hz, ArH), 6.79 (2H, d, *J=* 8.8 Hz, ArH), 7.01 (2H, d, *J=* 8.8 Hz, ArH), 7.21 (2H, d, *J=* 8.8 Hz, ArH), 9.64 (1H, br s, OH: exchanges with D₂O), 9.79 (1H, br s, OH: exchanges with D₂O).

### Preparation of 3,4-bis(4-methoxyphenyl)-5-methylisoxazole ¹¹C-(III)

To a solution of NaH (9.8 mg, 0.41mmol) in DMF (0.3mL) the compound 2 (50 mg, 0,19mmol) in DMF (0,4mL) was added dropwise. The mixture was stirred for 10 minutes at r.t. and then CH₃I (0.1 mL, 1.5 mmol) was added, to give a pale orange homogeneous solution that was stirred for a further 1h. The reaction crude was extracted with ethyl acetate three times and washed with brine three times, to remove DMF. The organic layer was dried over anhydrous Na₂SO₄ and the solvent removed under reduced pressure to give the white product (50% yield), which structure was confirmed by ¹H-NMR (CDCl₃) δ: 2.40 (3H, s), 3.83 (3H, s), 3.80 (3H, s) 6.83 (2H, d, J=9.0), 6.90 (2H, d, J=9.0 Hz) 7.09 (2H, d, J=9.0 Hz), 7.37 (2H, d, J=9.0 Hz). Mp: 95-98°C.

### Preparation of 5-bromomethyl-3,4-bis(4-methoxyphenyl)-isoxazole (V)

To a solution of (**III**) (500 mg, 1.7 mmol) in CCl₄ (8.75 mL) kept at r.t., NBS (362 mg, 2.05 mmol) and AIBN (140 mg, 0.85mmol) were added. The mixture was stirred overnight at r.t. to activate the substrate, giving a pale yellow homogeneous solution. After 16 h, the same amount of NBS and AIBN were added. The reaction mixture was refluxed for 2 hours, and then stopped by adding distilled water. The aqueous solution was extracted with CH₂Cl₂. The organic layer was dried over anhydrous Na₂SO₄ and the solvent removed under reduced pressure. The product (V) was isolated as a yellow solid (55% yield) by flash chromatography on silica gel (hexane/ethyl acetate 9:1) of the reaction crude, which chemical structure was assigned by ¹H NMR (400 MHZ, CDCl₃, δ): 3.80 (s, 3H, CH₃), 3.84 (s, 3H, CH₃); 4.44 (s, 2H, CH₂), 6.83 (d, 2H, J= 9.0 Hz, ArH), 6.93 (d, 2H, J= 9.0 Hz, ArH), 7.20 (d, 2H, J= 9.0 Hz, ArH); 7.38 (d, 2H, J= 9.0 Hz, ArH) and ESI-MS: *m*/*z* (%): C₁₇H₁₇BrNO₃ (M)⁺, 373.

### Preparation of 5-fluromethyl-3,4-bis(4-methoxyphenyl)-isoxazole (VI)

To 5-bromomethyl-3,4-bis(4-methoxyphenyl)-isoxazole **(V)** (50 mg, 0.134 mmol) at room temperature TBAF 1M in THF (402 µl) was added dropwise, and the mixture was stirred for 24 h at room temperature to give a pale red homogeneous solution. The reaction was stopped by addition of distilled water and the aqueous solution was extracted with ethyl acetate. The organic layer was dried over anhydrous Na₂SO₄ and the solvent removed under reduced pressure. The product **(VI)** was isolated as a yellow solid (45% yield) by column chromatography on silica gel (hexane/ethyl acetate 8:2) of the reaction crude, which the chemical structure was assigned by ¹H NMR (400 MHZ, CDCl₃, δ): 3.81 (s, 3H, CH₃), 3.84 (s, 3H, CH₃); 5.33 (d, 2H, J_{H-F} =48.4 Hz), 6.86 (d, 2H, J= 9.0 Hz ArH), 6.93 (d, 2H, J= 9.0 Hz, ArH), 7.17- (d, 2H, J= 9,0 Hz, ArH); 7.41 (d, 2H, J= 9.0 Hz, ArH) and ESI-MS: *m*/*z* (%): (M)⁺, 313.

### Preparation of 2-[3,4-bis(4-hydroxyphenyl)isoxazol-5-yl]acetic acid (VIII)

A mixture of mofezolac **6** (2.5 g, 7.4 mmol) and 47% HBr (10 mL) in AcOH (10 mL) was heated under reflux for 5h. Then, water was added and the product extracted with ethyl acetate. The organic layer was washed with water, dried over MgSO₄ and solvent removed under reduced pressure. Recrystallizion of the reaction crude gave **9** (0.8 g, 35%). Mp 183-185 °C (CH₃CN, dec., with foaming). IR: (KBr): 1720 (C=O) cm⁻¹. ¹H NMR (DMSO-*d₆*) δ: 3.75 (2H, s, CH₂), 6.75 (2H, d, *J*=8.8 Hz, ArH), 6.79 (2H, d, *J*=8.8 Hz, ArH), 7.01 (2H, d, *J*=8.8 Hz, ArH), 7.21 (2H, d, J=8.8 Hz, ArH), 9.64 (1H, br s, OH: exchanges with D₂O), 9.79 (1H, br s, OH: exchanges with D₂O), 12.89 (1H, br s, COOH: exchanges with D₂O). ¹³C-NMR (75 MHz, CD₃OD) δ: 31.3, 115.2, 115.5, 117.6, 119.9, 120.4, 157.6, 159.0, 161.3, 162.9, 170.4. Anal.Calcd for C₁₇H₁₃NO₅: C, 65.16; H, 4.40; N, 6.33. Found: C, 65.45; H, 4.38; N, 6.44.

### Preparation of methyl 2-[3,4-bis(4-methoxyphenyl)isoxazol-5-yl]-dimethylacetate (IX)

To a solution of NaH (9.8 mg, 0.6 mmol) in DMF (0.3 mL) **(VIII)** (50 mg, 0,19 mmol) in DMF (0.4mL) was added dropwise. The reaction mixture was stirred for 10 minutes and then CH₃I (1.5 mmol) was added and stirred for further 1h to give a pale orange homogeneous solution. The crude reaction was extracted with ethyl acetate three times, and washed with brine three times to remove mostly DMF. The organic layer was dried over anhydrous Na₂SO₄ and the solvent removed under reduced pressure. Chromatographic column of the reaction crude, afforded a white product **(IX)** (50% yield). ¹H NMR (CDCl₃) δ: 1.55 (6H, s), (3.44 (3H, s), 3.76 (3H, s), 3.82 (3H, s), 6.77 (2H, d, *J*= 9.0 Hz, ArH), 6.87 (2H, d, *J*= 9.0 Hz, ArH), 7.04 (2H, d, *J*= 9.0 Hz, ArH), 7.30 (2H, d, *J*= 9.0 Hz, ArH).

### Preparation of 3,4-bis(4-methoxyphenyl)-5-hydroxyethylisoxazole (X)

To a solution of mofezolac **(VII)** (200 mg, 0.589 mmol), in anhydrous THF (2 mL) kept at 0 °C by a bath of ice, 1M BMS in THF (1.16 mL, 1.16 mmol) was added dropwise, and the mixture was stirred overnight at room temperature to give a pale yellow homogeneous solution. Distilled water (1 mL), 20% NaOH (I mL) and 35% H₂O₂ (1 mL) were added and the obtained reaction mixture was stirred for 1 hour. Then, the solution was extracted with ethyl acetate. The organic layer was dried over anhydrous Na₂SO₄ and the solvent removed under reduced pressure. The product **(X)** was isolated as a white solid (68% yield) by flash chromatography on silica gel (hexane/ethyl acetate 6:4) of the reaction crude, the ¹H NMR (400 MHZ, CDCl₃, δ): 3.81 (s, 3H, CH₃), 3.85 (s, 3H, CH₃); 4.44 (t, 2H, J= 6.6 Hz CH₂), 4.26 (t, 2H, J= 6.6 Hz CH₂ 6.85 (d, 2H, J= 9.0 Hz, ArH), 6.95 (d, 2H, J= 9.0 Hz, ArH), 7.21 (d, 2H, J= 9.0 Hz, ArH); 7.39 (d, 2H, J= 9.0 Hz, ArH). ESI-MS: *m*/*z* (%): C₁₈H₂₀NO₄ (M)⁺, 325.

### Preparation of 2-[3,4-bis(4-methoxyphenyl)isoxazol-5-yl]ethyl-4-methylbenzenesulfonate (XI)

To a solution of **(X)** (3,4-bis(4-methoxyphenyl)-5-hydroxyethylisoxazole) (160 mg, 0.492 mmol), in CH₂Cl₂ (11.4 mL) kept at 0 °C by a bath ice, *p*-toluensulphonic anhydride (544 mg, 1.67 mmol) and triethylamine (466 µl) were added. The reaction mixture was stirred overnight at r.t. to give a pale yellow homogeneous solution. Then, water was added to the reaction mixture and the aqueous solution was extracted with CH₂Cl₂. The organic layer was dried over anhydrous Na₂SO₄ and the solvent removed under reduced pressure. The product (**XI**) was isolated as a yellow solid (60% yield) by flash chromatography on silica gel (hexane/ethyl acetate 7:3) of the reaction crude. ¹H NMR (400 MHZ, CDCl₃, δ): 2.36 (s, 3H, CH₃), 3.05 (t, 2H, J= 6.6 Hz CH₂); 3.78 (s, 3H, CH₃), 3.82 (s, 3H, CH₃), 4.30 (t, 2H, J= 6.6 Hz CH₂6.82 (d, 2H, J= 9.0 Hz, ArH), 6.88 (d, 2H, J= 9.0 Hz, ArH), 7.03 (d, 2H, J= 9.0 Hz, ArH); 7.30 (d, 2H, J= 9.0 Hz, ArH), 7.33 (d, 2H, J= 9.0 Hz, ArH), 7.69 (d, 2H, J= 9.0 Hz, ArH). ESI-MS: *m*/*z* (%): C₂₅H₂₅SNO₆ (M)⁺, 479.

### Preparation 3,4-bis(4-methoxyphenyl)-5-vinylisoxazole (XII)

To **(XI)** (30 mg, 0.063 mmol) TBAF 1M in THF (188 µL) was added dropwise. The reaction mixture was stirred for 24 h at room temperature to give a pale yellow homogeneous solution. The reaction was stopped by adding distilled water and product extracted with ethyl acetate. The organic layer was dried over anhydrous Na₂SO₄ and the solvent removed under reduced pressure. The product **(XII)** was isolated as a yellow solid (55% yield) by chromatography on silica gel (hexane/ethyl acetate 8:2) of the reaction crude. ¹H NMR (400 MH_{Z}, CDCl₃, δ): 5.54 (dd, 1H, J= 1.1 and 11.5 Hz, vinyl proton); 6.10 (dd, 1H, J= 1.1 and 17.6 Hz, vinyl proton); 6.52 (dd, 1H, J= 11.5 and 17.6 Hz, vinyl proton), 6.84 (d, 2H, J= 9.0 Hz, ArH), 6.92 (d, 2H, J= 9.0 Hz, ArH); 7.17 (d, 2H, J= 9.0 Hz, ArH), 7.39 (d, 2H, J= 9.0 Hz, ArH). ESI-MS: *m*/*z* (%): C₂₅H₂₅SNO₆ (M)⁺, 261.

### Preparation of 3-(5-chlorofuran-2-yl)-4-phenylisoxazol-5-amine (XIII)

A 2.5 M solution of n-butyllithium in hexane (1.068 mL, 2.67 mmol) was added to diisopropylamine (0.413 mL, 2.97 mmol) in anhydrous THF (10 mL) at 0°C under nitrogen atmosphere, using a nitrogen-flushed, three necked flask equipped with a magnetic stirrer, a nitrogen inlet and two dropping funnels. After the mixture had been stirred for 15 min, the reaction mixture was kept at -78 °C, then phenylacetonitrile (0.300 mL, 2.23 mmol) was dropwise added. The yellow reaction mixture was stirred at 0 °C for 1 h, then the solution of 5-chlorofuran-2-carbonitrile oxide (2.23 mmol) in anhydrous THF (10 mL) was added. The orange-colored reaction mixture was allowed to reach room temperature and stirred overnight. After quenching by addition of aqueous NH₄CI solution, the reaction products were extracted three times with ethyl acetate. The organic phase was dried over anhydrous Na₂SO₄ and then evaporated under vacuum. Column chromatography (silica gel, petroleum ether: ethyl acetate = from 20/1 to 8/2) of the residue affords the 5-(chlorofuran-2-yl)-4-phenylisoxazol-5-amine (105 mg) in 40% yield. Mp 138.5-141 °C (EtOAc/hexane). FT-IR (KBr): 3460, 3402.6, 3115, 2927, 1643, 1518, 1506, 1474, 1413, 1318, 1208, 1148, 1020, 988, 940, 896, 786, 699 cm⁻¹. ¹H NMR (400 MHz, CDCl₃, δ): 7.45-7.41 (m, 2H,-aromatic protons); 7.39-7.31(m, 3H, aromatic protons); 6.36 (d, J = 3.5 Hz, 1H, furyl proton); 6.14 (d, J = 3.5 Hz, 1H, furyl proton); 4.56 (bs, 2H, NH₂: exchange with D₂O). ¹³C NMR (100 MHz, CDCl3, δ): 165.8, 153.1, 143.9, 138.4, 130.01, 129.8, 129.3, 128.1, 113.7, 108.1, 93.5. GC-MS (70 eV) m/z (rel.int.): 262 [M (³⁷Cl)⁺, 11], 260 [M (³⁵Cl)⁺, 31], 225 (6), 218 (20), 216 (54),197 (8),188 (13),180 (20), 154 (15),153 (16),152 (32),131 (31),129 (100),127 (16),115 (10),113 (21), 105 (28), 104 (27), 103 (13), 101 (26), 94 (42), 89 (14), 85 1, 77 (54), 76 (11), 63 (15), 51 (14).

### Preparation of 3-(5-Chlorofuran-2-yl)-5-methyl-4-(4-nitrophenyl)isoxazole (XV)

A solution of the 1-(4-nitrophenyl)-2-propanone (0.6 mmol) in THF (3 mL) was added dropwise to a suspension of NaH (95% w/w, 1.2 mmol) in THF (6 mL) at 0 °C under nitrogen atmosphere, using a nitrogen-flushed, three necked flask equipped with a magnetic stirrer, a nitrogen inlet and two dropping funnels. After the yellow mixture had been stirred for one hour, a solution of 5-chlorofuran-2-carbonitrile oxide (0.6 mmol) in THF (3 mL) was added. The reaction mixture was allowed to reach room temperature, stirred overnight and then quenched by adding aqueous NH₄CI solution. The reaction products were extracted three times with ethyl acetate. The combined organic phases were dried over anhydrous Na₂SO₄ and then evaporated under vacuum. Column chromatography (silica gel, petroleum ether : ethyl acetate= 19/1) of the residue affords the product in 24 % yield. Mp 153-155 °C (hexane). White powder. FT-IR (KBr): 3144, 3103, 3071, 2929, 2851, 1628, 1601, 1559, 1519, 1441, 1419, 1345, 1239, 1204, 1136, 1105, 1019, 988, 896, 867, 853, 797, 761, 730, 709, 688, 560, 513 cm-1. ¹H NMR (400 MHz, CDCl₃, δ): 8.32 (d, 2H, J= 8.3 Hz, aromatic protons); 7.49 (d, 2H, J= 8.3 Hz, aromatic protons); 6.49 (d, 1H, J= 3.5 Hz); 6.21 (d, 1H, J= 3.5 Hz); 2.45 (s, 3H). ¹³C NMR (100 MHz, CDCl₃, δ): 167.7, 151.8, 147.6, 142.7, 138.8, 136.5, 130.8, 123.9, 113.7, 113.3, 108.1, 11.4. GC-MS (70 eV) *m*/*z* (int.rel.): 306 (M(37Cl)+, (15), 304 (M(35Cl)+, (45), 264 (33), 262 (100), 245 (7), 217 (13), 215 (15), 199 (7), 188 (9), 187 (12), 152 (9), 89 (16), 73 (8), 63 (6), 43 (52).

### Preparation of 4-(3-(5-chlorofuran-2-yl)-5-methylisoxazol-4-yl)benzenamine (XIV)

To a stirred mixture of stannous chloride (298 mg, 1.32 mmol) in hydrochloric acid 37% (1 mL) at 25 °C, was added a solution of 3-(5-chlorofuran-2-yl)-5-methyl-4-(4-nitrophenyl)isoxazole (**XV**) (100 mg, 0.33 mmol) dissolved in absolute EtOH (8 mL). The reaction mixture was kept under reflux for 4 h. 10% NaOH (10 mL) was added to the reaction mixture till pH 12, and the aqueous phase extracted three times with EtOAc. The combined organic extracts were dried over anhydrous Na2SO4 and the solvent removed under reduced pressure. The residue afforded the product with 98% yield. Mp 132-134 °C (EtOAc/hexane). FT-IR (KBr): 3486, 3386, 3143, 3032, 2924, 1626, 1520, 1441, 1411, 1298, 1234, 1204, 1180, 1136, 1016, 988, 941, 899, 838, 799, 739, 565, 518 cm⁻¹. ¹H NMR (300 MHz, CDCl3, δ): 7.26 (bs, 2H, NH₂: exchange with D₂O); 7.04 (d, 2H, J = 8.4 Hz, aromatic protons); 6.74 (d, 2H, J = 8.4 Hz, aromatic protons); 6.28 (d, 1H, J = 3.5 Hz, furyl proton); 6.13 (d, 1H, J = 3.5 Hz, furyl proton); 2.35 (s, 3H). ¹³C NMR (100 MHz, CDCl₃, δ): 166.8, 152.7, 146.8, 144.1, 138.4, 131.2, 119.1, 115.4, 114.9, 113.8, 108.1, 11.4. GC-MS (70 eV) m/z (rel.int.): 276 [M (³⁷Cl)⁺, 34], 274 [M (³⁵Cl)⁺, 100], 234 (13), 233 (19), 232 (40), 231 (48), 203 (15), 169 (19),168 (29),159 (26),144 (29),142 (12),119 (29),118 (21),117 (10), 113 (10), 89 (11), 77 (7), 65 (6), 63 (6), 51 (4), 43 (14).

## Claims

1. A compound selected from compounds of formula (V), (VI), (IX) and (XII) 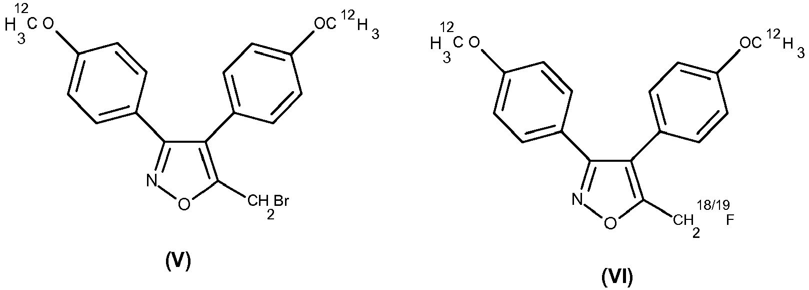 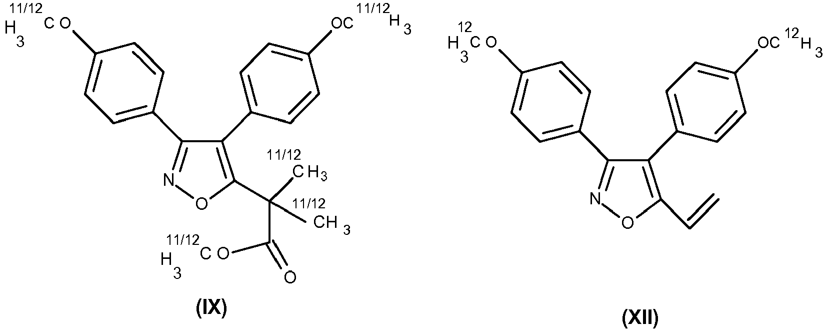 and their salts.

2. The compound according to claim 1, wherein said salts are pharmaceutically acceptable salts.

3. The compound of claim 1 or 2, which is selected from compounds of formula [18F]-(VI), [11C]-(IX) and their salts.

4. A pharmaceutical or diagnostic composition comprising at least one compound of claims 1 to 3, or pharmaceutically acceptable salts thereof.

5. The compounds of claims 1 to 3, or pharmaceutically acceptable salts thereof for use for preventing and/or treating a pathology selected from cancer, neuro-inflammation, inflammatory syndrome, cardioprotection, fever and pain.

6. Radiolabeled compounds according to claims 1 to 3, or pharmaceutically acceptable salts thereof for use in the diagnosis of neuro-inflammation and cancer.

7. The compounds for use according to claim 6, wherein said cancer is ovarian cancer.

8. The compounds for use according to claim 6 or 7, wherein said diagnosis is PET diagnosis.

## Patentansprüche

1. Verbindung, ausgewählt aus den Verbindungen der Formeln (V), (VI), (IX) und (XII) 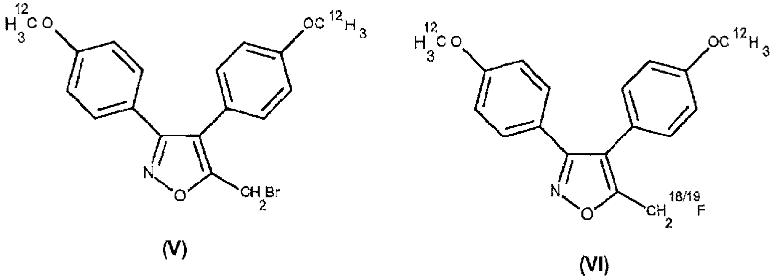 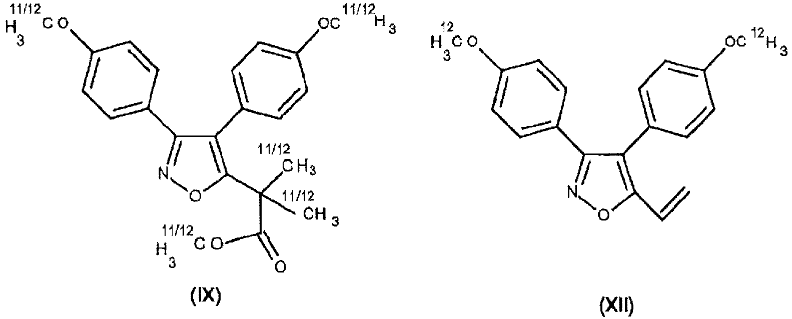 und deren Salzen.

2. Verbindung gemäß Anspruch 1, wobei die Salze pharmazeutisch annehmbare Salze sind.

3. Verbindung gemäß einem der Ansprüche 1 oder 2, welche ausgewählt ist aus Verbindungen der Formel [18F]-(VI), [11C]-(IX) und deren Salzen.

4. Pharmazeutische oder diagnostische Verbindung, umfassend mindestens eine Verbindung der Ansprüche 1 bis 3, oder pharmazeutisch annehmbare Salze davon.

5. Verbindungen der Ansprüche 1 bis 3, oder pharmazeutisch annehmbare Salze davon, zur Verwendung für die Vorbeugung und/oder die Behandlung einer Pathologie, die aus Krebs, Neuroinflammation, entzündlichem Syndrom, Kardioprotektion, Fieber und Schmerz ausgewählt ist.

6. Radiomarkierte Verbindungen gemäß einem der Ansprüche 1 bis 3, oder pharmazeutisch annehmbare Salze davon, zur Verwendung in der Diagnose von Neuroinflammation und Krebs.

7. Verbindung zur Verwendung gemäß Anspruch 6, wobei der Krebs ein Eierstockkrebs ist.

8. Verbindungen zur Verwendung gemäß einem der Ansprüche 6 oder 7, wobei die Diagnose eine PET-Diagnose ist.

## Revendications

1. Composé sélectionné à partir des composés de formule (V), (VI), (IX) et (XII) 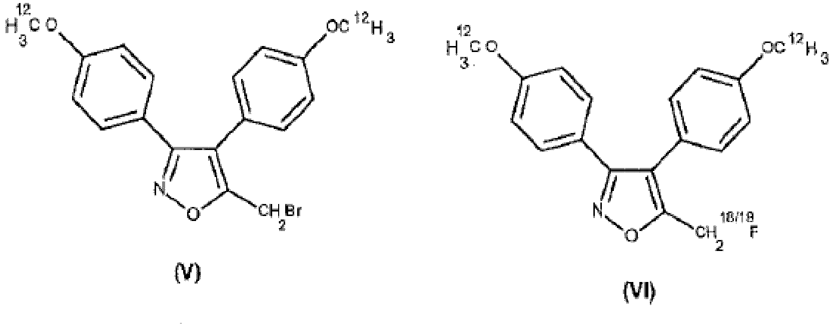 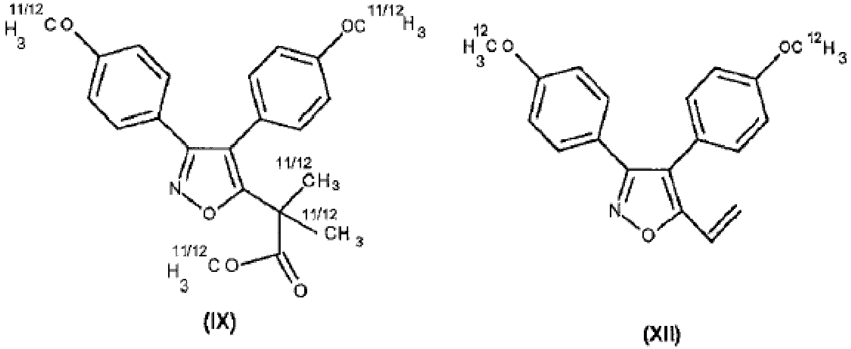 et leurs sels.

2. Composé selon la revendication 1, dans lequel lesdits sels sont des sels pharmaceutiquement acceptables.

3. Composé selon la revendication 1 ou 2, qui est sélectionné à partir des composés de formule [18F]-(VI), [11C]-(IX) et de leurs sels.

4. Composition pharmaceutique ou de diagnostic comprenant au moins un composé selon les revendications 1 à 3, ou des sels pharmaceutiquement acceptables de celui-ci.

5. Composés selon les revendications 1 à 3, ou des sels pharmaceutiquement acceptables de ceux-ci pour une utilisation pour prévenir et/ou traiter une pathologie sélectionnée à partir d'un cancer, d'une neuro-inflammation, d'un syndrome inflammatoire, d'une cardioprotection, d'une fièvre et d'une douleur.

6. Composés radiomarqués selon les revendications 1 à 3, ou les sels pharmaceutiquement acceptables de ceux-ci pour une utilisation dans le diagnostic d'une neuro-inflammation et d'un cancer

7. Composés pour une utilisation selon la revendication 6, dans lesquels ledit cancer est un cancer des ovaires.

8. Composés pour une utilisation selon la revendication 6 ou 7, dans lesquels ledit diagnostic est un diagnostic PET.
